(19) Europäisches Patentamt
European Patent Office
Office européen des brevets

(11) Publication number: **0 072 620**
**B1**

(12) **EUROPEAN PATENT SPECIFICATION**

(45) Date of publication of patent specification: **04.12.85**

(51) Int. Cl.⁴: **C 07 D 405/12,**
C 07 D 451/02,
C 07 D 453/02, A 61 K 31/445

(21) Application number: **82303634.8**

(22) Date of filing: **09.07.82**

(54) **3-Methylflavone-8-carboxylic acid esters.**

(30) Priority: **17.07.81 GB 8122158**

(43) Date of publication of application:
**23.02.83 Bulletin 83/08**

(45) Publication of the grant of the patent:
**04.12.85 Bulletin 85/49**

(84) Designated Contracting States:
**AT BE CH DE FR LI NL SE**

(56) References cited:
**US-A-2 921 070**
**US-A-4 217 351**

(73) Proprietor: **RECORDATI S.A. CHEMICAL and PHARMACEUTICAL COMPANY**
**Corso S. Gottardo 54**
**CH-6830 Chiasso (CH)**

(72) Inventor: **Nardi, Dante**
**Via T.Gulli 47**
**Milan (IT)**
Inventor: **Tajana, Alberto**
**Via Omboni 3**
**Milan (IT)**
Inventor: **Cazzulani, Pietro**
**Via F. Cavallotti 112**
**Casal Pusterlengo Milan (IT)**
Inventor: **Graziani, Gabriele**
**Via Dei Platani 8**
**Arese Milan (IT)**
Inventor: **Pennini, Renzo**
**Via Ricciarelli 8**
**Milan (IT)**
Inventor: **Casadio, Silvano**
**Via Tantardini 15**
**Milan (IT)**

(74) Representative: **SERJEANTS**
**25 The Crescent King Street**
**Leicester, LE1 6RX (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 072 620 B1

**Description**

The invention relates to esters of 3-methylflavone-8-carboxylic acid, to pharmaceutically acceptable salts thereof, to processes for the preparation of the esters and their salts, and to pharmaceutical compositions containing the esters or their salts. It is known that certain esters of 3-methylflavone-8-carboxylic acid exhibit a good spasmolytic activity (see US—A—2,921,070). Their action, however, might be improved by increasing their stability at physiological pH.

The invention provides 3-methylflavone-8-carboxylic acid esters having the general formula I

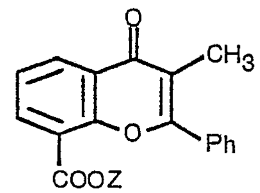

(I)

wherein Z represents an N-methylpiperidyl, tropyl or quinuclidyl group or a group of the general formula II

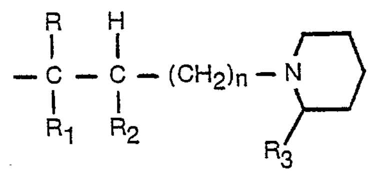

(II)

in which *n* is 0 or 1, R represents a hydrogen atom, an alkyl group having from 1 to 4 carbon atoms or a phenyl group, $R_1$ represents a hydrogen atom or an alkyl group having from 1 to 4 carbon atoms, $R_2$ represents a hydrogen atom or a hydroxy group, or R, $R_1$ and $R_2$ together with the carbon atoms to which they are attached represent a cycloalkyl ring having from 4 to 6 carbon atoms, and $R_3$ represents a hydrogen atom or an alkyl group having from 1 to 4 carbon atoms, with the proviso that R, $R_1$, $R_2$ and $R_3$ do not all simultaneously represent hydrogen atoms; and further provides pharmaceutically acceptable salts of such esters.

The compounds according to the invention have been found to exhibit powerful smooth muscle relaxant, calcium blocking, local anaesthetic, and anti-inflammatory properties. They are considerably stable at the physiological pH so that the half-life of the drug is highly prolonged. It has been found, moreover, that the novel esters possess further activities and that some of them have diminished toxicity when compared with the known compounds.

The invention further provides a process for the preparation of the compounds having the general formula I as above defined, the process comprising condensing a 3-methylflavone-8-carboxylic acid halide having the general formula III

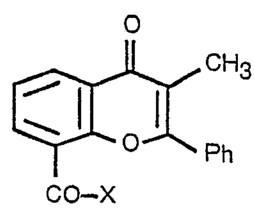

(III)

wherein X represents a halogen atom with an alcohol of the general formula ZOH, wherein Z is as above defined.

The 3-methylflavone-8-carboxylic acid halide is preferably 3-methylflavone-8-carboxylic acid chloride (III, X=Cl), and is known compound (see US—A—2921070). It is readily prepared by reacting the corresponding acid with thionyl chloride or phosphorus tirchloride. The acid is prepared according to the standard procedures for the manufacturing of flavones.

The condensation may be carried out in the presence or absence of a solvent. If carried out in the absence of a solvent, the reactants are heated together at a temperature of from 140°C to 200°C, and an excess of the 3-methyl-flavone-8-carboxylic acid is employed. If carried out in the presence of a solvent, the reactants are generally used in equimolar proportions, the temperature may be from 0°C to the reflux temperature of the solvent, and an acid-binding agent (hydrogen halide acceptor) may optionally be present. Suitable solvents include all inert organic solvents, particularly dimethylformamide, ethers and halogenated hydrocarbons. Aromatic hydrocarbons, such as benzene and toluene, are also useful,

especially when the reaction is carried out at reflux temperature. The acid-binding agent may be any of those customarily used in such condensations, for example, organic bases, such as triethylamine and inorganic bases such as alkali metal hydroxides and alklai metal carbonates.

The invention also provides a further process for the preparation of the compounds having the general formula I in which Z represents a group of the general formula II wherein n is 1, R and $R_1$ both represent hydrogen atoms and $R_2$ represents a hydroxy group. This further process comprises reacting a compound of the general formula IV

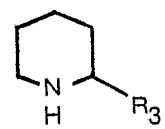

(IV)

wherein $R_3$ is as above defined with 2,3-epoxypropyl 3-methylflavone-8-carboxylate in the presence of a catalyst. The 2,3-epoxypropyl 3-methylflavaone-8-carboxylate is a novel compound, but can be prepared by condensing a 3-methylflavone-8-carboxylic acid halide (preferably the chloride) with 2,3-epoxy-1-propanol. The reaction may be carried out in the presence of an organic solvent, such as any of those previously mentioned or a nitrile such as acetonitrile. The catalyst may be an organic base such as triethylamine.

Generally equimolar amounts of reactants are employed, and the temperature ranges from 20 to 80°C. The reaction is preferably carried out at 40—60°C.

The salts according to the invention may be prepared from the basic esters obtained by the processes described above according to conventional methods such as addition of an acid to the free base dissolved in a suitable solvent. Suitable acids include hydrogen halides, phosphoric acid, nitric acid, alkylsulphonic acids, arylsulphonic acids, monofunctional and bifunctional carboxylic acids, hydroxycarboxylic acids and 1,5-naphthalenedisulphonic acid. Isolation and purification may be effected conventionally.

The invention additionally provides a pharmaceutical composition comprising a compound having the general formula I as above defined or a pharmaceutically acceptable salt thereof in admixture with a pharmaceutically acceptable diluent or carrier.

The active compounds according to the invention exhibit a powerful smooth muscle relaxant and a calcium blocking action. They are also good anaesthetic and anti-inflammatory agents. As stated above, their stability at physiological pH is, however, the most interesting factor when compared with similar flavone derivatives. The stability was studied at 37°C in gastric fluid simulated (U.S.P. XX, 1105, 1980) and in phosphate buffer (pH 7.4) by spectrodensitometric determination of 3-methylflavone-8-carboxylic acid resulting from eventual hydrolysis. As comparison substance 2-piperidino-ethyl-3-methylflavone-8-carboxylate hydrochloride (Flavoxate) was selected, this being the best of the esters disclosed in US—A—2,921,070.

The results are reported in Table 1, in which the figures in the columns headed "1 hour" and "3 hours" represent the amount of the active compound remaining in the buffered simulated gastric fluid after the specified times expressed as a percentage of the amount of active compound originally in the buffered simulated gastric fluid. In this Table, and in subsequent Tables, the numbers identifying the active compounds are those assigned to the respective active compounds in the Examples which follow and which describe their preparation.

TABLE 1
Stability test, pH 7.4

| Active compound | 1 hour | 3 hours |
|---|---|---|
| 1 | 100 | 100 |
| 2 | 100 | 100 |
| 3 | 40 | 30 |
| 4 | 68 | 34 |
| 5 | 96 | 91 |
| 6 | 100 | 100 |
| 7 | 44 | 25 |
| 8 | 85 | 70 |
| 9 | 88 | 87 |
| 10 | 100 | 100 |
| 11 | 100 | 100 |
| 12 | 100 | 100 |
| 13 | 100 | 100 |
| FLAVOXATE | 22 | 10 |

The $LD_{50}$ of the new esters was determined in mice both i.p and per os, following the method described by C.S. Weil (Biometrics, *8*, 249, 1952). The results obtained are reported in Table II.

TABLE II
$LD_{50}$ mM/Kg

| Active compound | i.p. | os |
|---|---|---|
| 1 | 0.56 | 1.86 |
| 2 | 0.16 | 0.69 |
| 3 | 0.5 | 0.85 |
| 7 | 0.58 | 3.10 |
| 8 | — | 2.16 |
| 9 | 0.19 | 1.08 |
| 10 | 0.15 | 0.85 |
| 11 | 0.36 | 0.77 |
| 12 | 0.42 | 0.95 |
| 13 | 0.12 | 0.50 |
| FLAVOXATE | 0.90 | 1.89 |

The calcium blocking activity was tested on guinea pig depolarized taenia coli, according to the method described by Ferrari and Carpenedo (Arch. Int. Pharmacodyn., *174*, 223, 1968). The guinea pig taenia coli was allowed to stabilize in Tyrode solution without $Ca^{++}$. It was then washed with $K_2SO_4$ Ringer solution and afterwards perfused with $KNO_3$ Ringer. Cumulative concentrations of $CaCl_2$ were added to the organ bath in absence or presence of the drug to test. The obtained results are reported in Table III.

TABLE III
Calcium blocking activity

| Active compound | $ED_{50}$ (μM) |
|---|---|
| 1 | 24 |
| 2 | 16 |
| 3 | 19 |
| 5 | 8.6 |
| 6 | 7.5 |
| 8 | 7.3 |
| 9 | 26 |
| FLAVOXATE | 25 |

The antispastic activity was evaluated following the Magnus method (Pflügers Arch. Gen. Physiol., *102*, 123, 1904). Two equal contractions were induced by $BaCl_2$ at a concentration ranging between 1 and $4.10^{-4}$ M in guinea pig ileum maintained at 30°C in Ringer solutin and aerated with Carbogen.

The drug to test was administered and, a minute later, $BaCl_2$ to the same concentration. The inhibition of the contractions was observed. The results are given in Table IV.

TABLE IV

| Active compound | $ED_{50}$ (μM) |
|---|---|
| 1 | 4 |
| 2 | 6.2 |
| 3 | 9.7 |
| 4 | 6.7 |
| 5 | 2.4 |
| 6 | 3.4 |
| 8 | 2.7 |
| 9 | 4 |
| 12 | 4.3 |
| 13 | 2.1 |
| 14 | 7.7 |
| FLAVOXATE | 5.6 |

The smooth muscle relaxant activity of the novel flavone derivatives has been also studied through the spontaneous mobility of guinea pig isolated ureter. The test has been executed according to the

Trendelemburg method (Arch.Exp.Path.Pharmak., *81*, 55, 1917). The ureter was allowed to stabilize in Tyrode solution with the upper part closed and the inner part connected to a pressure transducer. The drugs to be tested were given in a cumulative way and the spontaneous circular and longitudinal contractions of the ureter were measured. The results obtained are listed in Table V.

TABLE V
Antispastic activity Isolated ureter ($ED_{50}$,µM)

| Active compound | circular contractions | longitudinal contractions |
|---|---|---|
| 1 | 8.5 | 6.9 |
| 5 | 12 | 2.8 |
| 6 | 5.3 | 2.5 |
| 8 | 5 | 2.5 |
| 12 | 20 | 9 |

The following Examples illustrate the invention.

Example 1

To a mixture consisting of 7.86 g of 3-(2-methylpiperidino)-propanol, 40 ml of anhydrous dimethylformamide and 10.5 g of anhydrous potassium carbonate, 14.9 g of 3-methyl-flavone-8-carboxylic acid chloride were added. The mixture was heated at 60°C for 8 hours under stirring then it was poured in 500 ml of icy water. The precipitate thus formed was extracted with diethyl ether, washed with water and dried. The solvent was evaporated off and the compound, as brown oil residue, was transformed into the corresponding hydrochloride (1) by adding hydrogen chloride in isopropanol. The 3-(2-methylpiperidino)-propyl 3-methylflavone-8-carboxylate hydrochloride melts at 185—187°C.

Example 2

To a stirred suspension consisting of 5.75 g of 3-hydroxy-N-methylpiperidine and 10.4 g of anhydrous potassium carbonate in 50 ml dimethylformamide, 14.9 g of 3-methylflavone-8-carboxylic acid chloride were added. The mixture was stirred at ambient temperature for 20 hours, poured into icy water and the precipitate thus formed was extracted with ethyl acetate. The extract was neutralized with water and dried. The solvent was evaporated off and the residue was dissolved in isopropanol. After cooling, hydrogen chloride in isopropanol was added. The hydrochloride (11) was washed with diethyl ether and crystallized from isopropanol. The desired N-methyl-3-piperidyl 3-methylflavone-8-carboxylate hydrochloride melts at 228—229°C. The free base was crystallised from hexane, mp 90—93°C.

Example 3

To a stirred mixture, cooled at 10—15°C, and consisting of 12.8 g of 3-hydroxyquinuclidine, 240 ml of dimethylformamide and 20 g of triethylamine, were added in 30—40 minutes 29.8 g of 3-methylflavone-8-carboxylic acid chloride. The temperature was allowed to reach 20—25°C, the mixture was stirred for 4 hours and then poured into icy water. The precipitate thus formed was separated, washed with water and then extracted with ethyl acetate. The extract was washed with aqueous sodium carbonate solution, then with water and dried. The residue was chromatographed on a silica gel column using ethyl acetate: methanol (7:3 by volume) and chloroform: methanol (87:13 by volume) as eluent. 11.25 g of the desired compound, mp 180—181°C, were obtained. The free base was transformed into the corresponding hydrochloride (13) by adding hydrogen chloride in ethanol. The 3-quinuclidyl 3-methylflavone-8-carboxylate hydrochloride was crystallised from ethanol, mp 302—305°C.

Example 4

To a solution of 4.6 g of cis-2-piperidino-cyclohexanol in 100 ml of anhydrous benzene, stirred and maintained at 20—25°C, 7.5 g of 3-methylflavone-8-carboxylic acid chloride were added over a period of 15 minutes. The mixture was refluxed for 18 hours, then cooled to ambient temperature. The product thus formed was filtered off, washed with ethyl acetate, dried, and crystallized from ethanol. The cis-2-piperidino-cyclohexyl 3-methyl-flavone-8-carboxylate hydrochloride (8) melts at 258—259°C. The trans form (14) was similarly obtained by starting from trans-2-piperidino-cyclohexanol. Its hydrochloride melts at 222—225°C. Using 1,1-dimethyl-2-piperidino-ethanol instead of cis-2-piperidino-cyclohexanol, 1,1-dimethyl-2-piperidino-ethyl 3-methylflavone-8-carboxylate was prepared. The compound was directly isolated as its hydrochloride (5) of melting point 203—207°C.

From this salt the free base was separated. It melted at 103—105°C. Moreover, the following salts have been prepared:

| | |
|---|---|
| Nitrate | mp. 179°C (with decomposition) |
| Sulphate | mp. 208°C (with decomposition) |
| Phosphate | mp. 175—176°C (with decomposition) |
| Maleate | mp. 144—148°C |
| *p*-Toluenesulphonate | mp. 149—152°C |

Using 1,1-dimethyl-2-(2-methylpiperidino)-ethanol instead of cis-2-piperidino-cyclohexyl, 1,1-dimethyl-2-(2-methyl-piperidino)-ethyl 3-methylflavone-8-carboxylate (6) was obtained. This compound, as hydrochloride, melts at 194—195°C.

The free base was separated from this salt, and had a melting point of 78—80°C. The following salts were also prepared:

| | |
|---|---|
| Hydrobromide | mp. 209°C |
| Nitrate | mp. 170°C (with decomposition) |
| Sulphate | mp. 184—188°C |
| Maleate | mp. 147—149°C |
| *p*-Toluenesulphonate | mp. 157—159°C |

### Example 5

A mixture comprising 8.9 g of tropine hydrochloride and 22.5 g of 3-methylflavone-8-carboxylic acid chloride was heated for 4 hours at 170—175°C under a nitrogen atmosphere. When the reaction was over, a further 7.5 g of the flavone derivative were added and the mixture was heated again for 11 hours at the same temperature. After cooling, the product was powdered, suspended in water ans stirred for 6 hours. The whole was filtered off and dilute sodium hydroxide solution was added to the solution. The precipitate was centrifuged, washed with water and dried. The desired compound, tropyl 3-methyl-flavone-8-carboxylate, was treated with hydrogen chloride in ethanol to give the corresponding hydrchloride (12), melting point 276—278°C.

### Example 6

21.3 g of 3-methylflavone-8-carboxylic acid chloride were slowly added, over a period of 4 hours at 20°C, to a stirred solution of 5.55 g of 2,3-epoxy-1-propanol and 8.34 g of triethylamine in 165 ml of anhydrous benzene. The mixture was allowed to stand at 20—25°C for 20 hours, and then 60 ml of water were added. The whole was stirred for 15 minutes and the layers were separated. The organic layer was washed and dried, the solvent was evaporated off and 18.95 g of 2,3-epoxypropyl 3-methylflavone-8-carboxylate, m.p. 103—105°C, was obtained.

A mixture comprising 16.8 g of the 2,3-epoxypropyl 3-methylflavone-8-carboxylate, 100 ml of acetonitrile, 5.5 ml of piperidine and 7 ml of triethylamine was heated at 60°C for 12 hours. The solvent was evaporated off and the brown, oily residue was washed twice with 75 ml of benzene. The 2-hydroxy-3-piperidino-propyl 3-methyl-flavone-8-carboxylate thus formed was converted into the corresponding hydrochloride (7) in the usual manner. Mp 187—189°C.

### Example 7

Following the procedure described in Example 2, the following esters of 3-methylflavone-8-carboxylic acid were prepared from appropriate starting materials:

(2) 1-methyl-3-piperidino-propyl, separated as its hydrochloride hemihydrate, mp 161—168°C;
(10) 1-methyl-4-piperidyl, melting at 103—105°C;
(3) 1-methyl-2-piperidino-ethyl, separated as its hydrochloride hydrate, mp 218—220°C;
(4) 1-phenyl-2-piperidino-ethyl (hydrochloride) of melting point 219—221°C; and
(9) 2-piperidinomethyl cyclohexyl, as hydrochloride, mp 236—237°C.

**Claims for the Contracting States: BE CH DE FR LI NL SE**

1. A 3-methylflavone-8-carboxylic acid ester having the general formula I

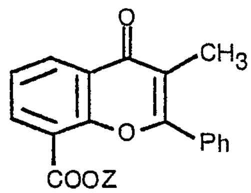

(I)

wherein Z represents an N-methylpiperidyl, tropyl or quinuclidyl group or a group of the general formula II

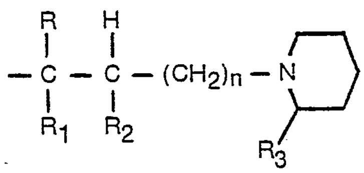

(II)

in which *n* is 0 or 1, R represents a hydrogen atom, an alkyl group having from 1 to 4 carbon atoms or a phenyl group, $R_1$ represents a hydrogen atom or an alkyl group having from 1 to 4 carbon atoms, $R_2$ represents a hydrogen atom or a hydroxy group, or R, $R_1$ and $R_2$ together with the carbon atoms to which they are attached represent a cycloalkyl ring having from 4 to 6 carbon atoms, and $R_3$ represents a hydrogen atom or an alkyl group having from 1 to 4 carbon atoms, with the proviso that R, $R_1$, $R_2$ and $R_3$ do not all simultaneously represent hydrogen atoms; or a pharmaceutically acceptable salt of such an ester.

2. 3-(2-Methylpiperidino)-propyl 3-methylflavone-8-carboxylate.
3. N-Methyl-3-piperidyl 3-methylflavone-8-carboxylate.
4. 3-Quinuclidyl 3-methylflavone-8-carboxylate.
5. cis-2-Piperidino-cyclohexyl 3-methylflavone-8-carboxylate.
6. trans-2-Piperidino-cyclohexyl 3-methylflavone-8-carboylate.
7. 1,1-Dimethyl-2-piperidino-ethyl 3-methylflavone-8-carboxylate.
8. 1,1-Dimethyl-2-(2-methylpiperidino)-ethyl 3-methylflavone-8-carboxylate.
9. Tropyl 3-methylflavone-8-carboxylate.
10. 2-Hydroxy-3-piperidinopropyl 3-methylflavone-8-carboxylate.
11. 1-Methyl-3-piperidino-propyl 3-methylflavone-8-carboxylate.
12. 1-Methyl-4-piperidyl 3-methylflavone-8-carboxylate.
13. 1-Methyl-2-piperidino-ethyl 3-methylflavone-8-carboxylate.
14. 1-Phenyl-2-piperidino-ethyl 3-methylflavone-8-carboxylate.
15. 2-Piperidinomethyl-cyclohexyl 3-methylflavone-8-carboxylate.
16. A process for the preparation of a compound according to claim 1, the process comprising condensing a 3-methylflavone-8-carboxylic acid halide having the general formula III

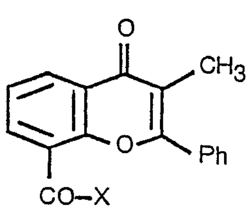

(III)

wherein X represents a halogen atom with an alcohol of the general formula ZOH wherein Z is as defined in claim 1.

17. A process according to claim 16 in which X respresents a chlorine atom.

18. A process according to claim 16 or claim 17 carried out by heating the alcohol with an excess of the flavone derivative at from 140°C to 200°C in the absence of a solvent.

19. A process according to claim 16 or claim 17 carried out by reacting the alcohol with an equimolar amount of the flavone derivative in the presence of a solvent at a temperature of from 0°C to the reflux temperature of the solvent.

20. A process according to claim 19 carried out in the presence of an acid-binding agent.

21. A process for the preparation of a 3-methylflavone-8-carboxylic acid ester having the general formula I

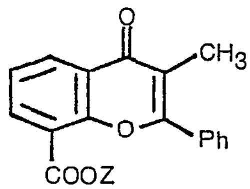

(I)

wherein Z represents a group of the general formula II

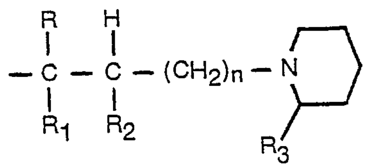

(II)

wherein *n* is 1, R and $R_1$ both represent hydrogen atoms, $R_2$ represents a hydroxy group and $R_3$ represents a hydrogen atom or an alkyl group having from 1 to 4 carbon atoms, the process comprising reacting a compound of the general formula IV

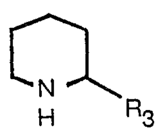

(IV)

wherein $R_3$ is as defined in claim 1 with 2,3-epoxypropyl 3-methylflavone-8-carboxylate in the presence of a catalyst.

22. A process according to claim 21 in which the catalyst is triethylamine.

23 A process according to claim 22 carried out in a solvent at from 20°C to 80°C using equimolar proportions of the flavone and piperidine derivatives.

24. A pharmaceutical composition comprising a 3-methylflavone-8-carboxylic acid ester according to claim 1 or a pharmaceutically acceptable salt of such an ester in admixture with a pharmaceutically acceptable diluent or carrier.

**Claims for the Contracting State: AT**

1. A process for the preparation of a 3-methylflavone-8-carboxylic acid ester having the general formula I

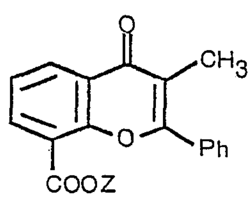

(I)

wherein Z represents an N-methylpiperidyl, tropyl or quinuclidyl group or a group of the general formula II

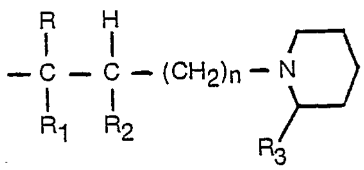

(II)

in which *n* is 0 or 1, R represents a hydrogen atom, an alkyl group having from 1 to 4 carbon atoms or a phenyl group, $R_1$ represents a hydrogen atom or an alkyl group having from 1 to 4 carbon atoms, $R_2$ represents a hydrogen atom or a hydroxy group, or R, $R_1$ and $R_2$ together with the carbon atoms to which they are attached represent a cycloalkyl ring having from 4 to 6 carbon atoms, and $R_3$ represents a hydrogen atom or an alkyl group having from 1 to 4 carbon atoms, with the proviso that R, $R_1$, $R_2$ and $R_3$ do not all simultaneously represent hydrogen atoms, the process comprising condensing a 3-methylflavone-8-carboxylic acid halide having the general formula III

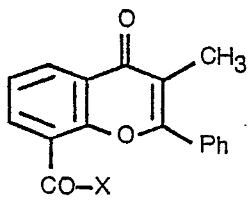

(III)

wherein X represents a halogen atom with an alcohol of the general formula ZOH wherein Z is as defined in this claim.

2. A process according to claim 1 in which X represents a chlorine atom..

3. A process according to claim 1 or claim 2 carried out by heating the alcohol with an excess of the flavone derivative at from 140°C to 200°C in the absence of a solvent.

4. A process according to claim 1 or claim 2 carried out by reacting the alcohol with an equimolar amount of the flavone derivative in the presence of a solvent at a temperature of from 0°C to the reflux temperature of the solvent.

5. A process according to claim 4 carried out in the presence of an acid-binding agent.

6. A process for the preparation of a 3-methylflavone-8-carboxylic acid ester having the general formula I

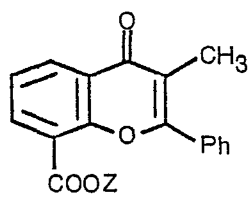

(I)

wherein Z represents a group of the general formula II

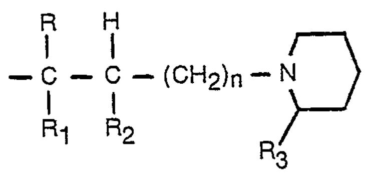

(II)

wherein *n* is 1, R and $R_1$ both represent hydrogen atoms, $R_2$ represents a hydroxy group and $R_3$ represents a hydrogen atom or an alkyl group having from 1 to 4 carbon atoms, the process comprising reacting a compound of the general formula IV

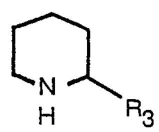

(IV)

wherein $R_3$ is as defined in claim 1 with 2,3-epoxypropyl 3-methylflavone-8-carboxylate in the presence of a catalyst.

7. A process according to claim 6 in which the catalyst is triethylamine.

8. A process according to claim 7 carried out in a solvent at from 20°C to 80°C using equimolar proportions of the flavone and piperidine derivatives.

**Patentansprüche für die Vertragsstaaten: BE CH LI DE FR NL SE**

1. Ein 3-Methylflavon-8-carbonsäureester der allgemeinen Formel I

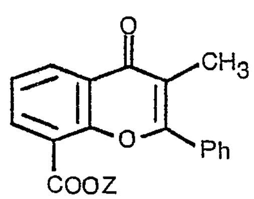

(I)

worin Z eine N-Methylpiperidyl-, Tropyl- oder Chinuclidylgruppe oder eine Gruppe der allgemeinen Formel II

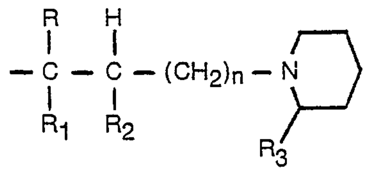

(II)

in welcher n 0 oder 1 ist, R ein Wasserstoffatom, eine Alkylgruppe mit 1 bis 4 Kohlenstoffatomen oder eine Phenylgruppe darstellt, $R_1$ ein Wasserstoffatom oder eine Alkylgruppe mit 1 bis 4 Kohlenstoffatomen darstellt, $R_2$ ein Wasserstoffatom oder eine Hydroxygruppe darstellt oder R, $R_1$ und $R_2$ zusammen mit dem Kohlenstoffatom, an welchem sie hängen, einen Cycloalkylring mit 4 bis 6 Kohlenstoffatomen darstellen und $R_3$ ein Wasserstoffatom oder eine Alkylgruppe mit 1 bis 4 Kohlenstoffatomen darstellt, bedeutet, mit der Bedingung, daß R, $R_1$, $R_2$ und $R_3$ nicht alle gleichzeitig Wasserstoffatome darstellen, oder ein pharmazeutisch brauchbares Salz eines solchen Esters.

2. 3-(2-Methylpiperidino)-propyl-3-methylflavon-8-carboxylat.
3. N-Methyl-3-piperidyl-3-methylflavon-8-carboxylat.
4. 3-Chinuclidyl-3-methylflavon-8-carboxylat.
5. cis-2-Piperidino-cyclohexyl-3-methylflavon-8-carboxylat.
6. trans-2-Piperidino-cyclohexyl-3-methylflavon-8-carboxylat.
7. 1,1-Dimethyl-2-piperidino-äthyl-3-methylflavon-8-carboxylat.
8. 1,1-Dimethyl-2-(2-methylpiperidino)-äthyl-3-methylflavon-8-carboxylat.
9. Tropyl-3-methylflavon-8-carboxylat.
10. 2-Hydroxy-3-piperidinopropyl-3-methylflavon-8-carboxylat.
11. 1-Methyl-3-piperidino-propyl-3-methylflavon-8-carboxylat.
12. 1-Methyl-4-piperidyl-3-methylflavon-8-carboxylat.
13. 1-Methyl-2-piperidino-äthyl-3-methylflavon-8-carboxylat.
14. 1-Phenyl-2-piperidino-äthyl-3-methylflavon-8-carboxylat.
15. 2-Piperidinomethyl-cyclohexyl-3-methylflavon-8-carboxylat.
16. Ein Verfahren zur Herstellung einer Verbindung nach Anpsruch 1, welches Verfahren das Kondensieren eines 3-Methylflavon-8-carbonsäurehalogenides der allgemeinen Formel III

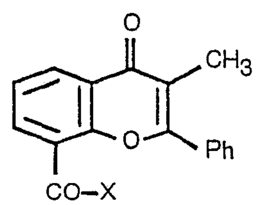

(III)

worin X ein Halogenatom darstellt, mit einem Alkohol der allgemeinen Formel ZOH, worin Z wie im Anspruch 1 festgelegt ist, umfaßt.

17. Ein Verfahren nach Anspruch 16, in welchem X ein Chloratom darstellt.

18. Ein Verfahren nach Anspruch 16 oder Anspruch 17, welches durch Erhitzen des Alkoholes mit einem Überschuß des Flavonderivates bei 140°C bis 200°C in Abwesenheit eines Lösungsmittels durchgeführt wurde.

19. Ein Verfahren nach Anspruch 16 oder 17, welches durch Umsetzen des Alkoholes mit einer äquimolaren Menge des Flavonderivates in Gegenwart eines Lösungsmittels bei einer Temperatur von 0°C bis zur Rückfluß-temperatur des Lösungsmittels durchgeführt wurde.

20. Ein Verfahren nach Anspruch 19, welches in Gegenwart eines säurebindenden Mittels durchgeführt wurde.

21. Ein Verfahren zur Herstellung eines 3-Methylflavon-8-carbonsäureesters der allgemeinen Formel I

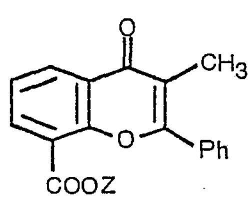

(I)

worin Z eine Gruppe der allgemeinen Formel II

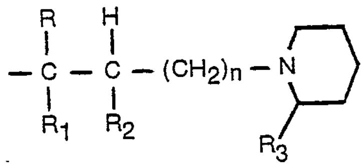

(II)

worin n 1 ist, R und $R_1$ beide Wasserstoffatome darstellen, $R_2$ eine Hydroxygruppe darstellt und $R_3$ ein Wasserstoffatom oder eine Alkylgruppe mit 1 bis 4 Kohlenstoffatomen darstellt, bedeutet, welches das Umsetzen einer Verbindung der allgemeinen Formel IV

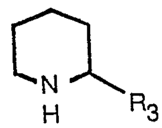

(IV)

worin $R_3$ wie im Anspruch 1 festgelegt ist, mit 2,3-Epoxypropyl-3-methylflavon-8-carboxylat in Gegenwart eines Katalysators umfaßt.

22. Ein Verfahren nach Anspruch 21, bei welchem der Katalysator Triäthylamin ist.

23. Ein Verfahren nach Anspruch 22, welches in einem Lösungsmittel bei 20°C bis 80°C unter Verwendung äquimolarer Anteile der Flavon- und Piperidinderivate durchgeführt wurde.

24. Eine pharmazeutische Zubereitung mit einem Gehalt an einem 3-Methylflavon-8-carbonsäureester nach Anspruch 1 oder einem pharmazeutisch brauchbaren Salze eines solchen Esters in Mischung mit einem pharmazeutisch brauchbaren Verdünnungsmittel oder Träger.

**Patentansprüche für den Vertragsstaat: AT**

1. Ein Verfahren zur Herstellung eines 3-Methylflavon-8-carbonsäureesters der allgemeinen Formel I

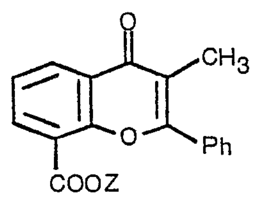

(I)

worin Z eine N-Methylpiperidyl-, Tropyl- oder Chinuclidylgruppe oder eine Gruppe der allgemeinen Formel II

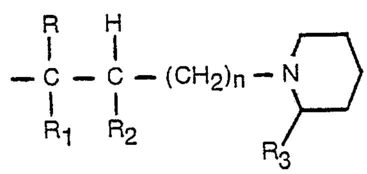

(II)

in welcher n 0 oder 1 ist, R ein Wasserstoffatom, eine Alkylgruppe mit 1 bis 4 Kohlenstoffatomen oder eine Phenylgruppe darstellt, $R_1$ ein Wasserstoffatom oder eine Alkylgruppe mit 1 bis 4 Kohlenstoffatomen darstellt, $R_2$ ein Wasserstoffatom oder eine Hydroxygruppe darstellt oder R, $R_1$ und $R_2$ zusammen mit den Kohlenstoffatom, an welchem sie hängen, einen Cycloalkylring mit 4 bis 6 Kohlenstoffatomen darstellen und $R_3$ ein Wasserstoffatom oder eine Alkylgruppe mit 1 bis 4 Kohlenstoffatomen darstellt, bedeutet, mit der Bedingung, daß R, $R_1$, $R_2$ und $R_3$ nicht alle gleichzeitig Wasserstoffatome darstellen, welches Verfahren das Kondensieren eines 3-Methylflavon-8-carbonsäure-halogenides der allgemeinen Formel III

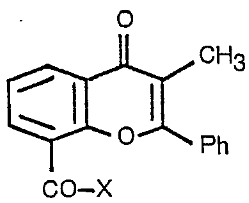

(III)

worin X ein Halogenatom darstellt, mit einem Alkohol der allgemeinen Formel ZOH, worin Z wie in diesem Anspruch 1 festgelegt ist, umfaßt.

2. Ein Verfahren nach Anspruch 1, in welchem X ein Chloratom darstellt.

3. Ein Verfahren nach Anspruch 1 oder Anspruch 2, welches durch Erhitzen des Alkoholes mit einem Überschuß des Flavonderivates bei 140°C bis 200°C in Abwesenheit eines Lösungsmittels durchgeführt wurde.

4. Ein Verfahren nach Anspruch 1 oder 2, welches durch Umsetzen des Alkoholes mit einer äquimolaren Menge des Flavonderivates in Gegenwart eines Lösungsmittels bei einer Temperatur von 0°C bis zur Rückflußtemperatur des Lösungsmittels durchgeführt wurde.

5. Ein Verfahren nach Anspruch 4, welches in Gegenwart eines säurebindenden Mittels durchgeführt wurde.

6. Ein Verfahren zur Herstellung eines 3-Methylflavon-8-carbonsäureesters der allgemeinen Formel I

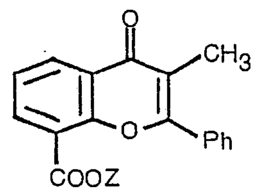

(I)

worin Z eine Gruppe der allgemeinen Formel II

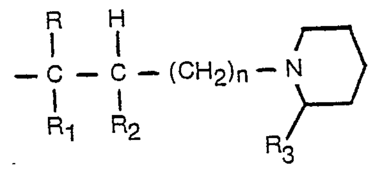

(II)

worin n 1 ist, R und $R_1$ beide Wasserstoffatome darstellen, $R_2$ eine Hydroxygruppe darstellt und $R_3$ ein Wasserstoffatom oder eine Alkylgruppe mit 1 bis 4 Kohlenstoffatomen darstellt, bedeutet, welches Verfahren das Umsetzen einer Verbindung der allgemeinen Formel IV

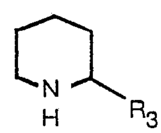

(IV)

worin $R_3$ wie im Anspruch 1 festgelegt ist, mit 2,3-Epoxypropyl-3-methylflavon-8-carboxylat in Gegenwart eines Katalysators umfaßt.

7. Ein Verfahren nach Anspruch 6, bei welchem der Katalysator Triäthylamin ist.

8. Ein Verfahren nach Anspruch 7, welches in einem Lösungsmittel bei 20°C bis 80°C unter Verwendung äquimolarer Anteile der Flavon- und Piperidinderivate durchgeführt wurde.

**Revendications pour les Etats Contractants: BE CH LI DE FR NL SE**

1. Ester de l'acide méthyle-3-flavone-carboxylique-8-répondant à la formule générale I

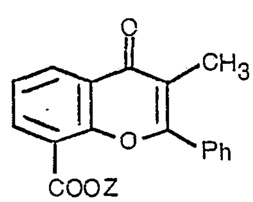

(I)

dans laquelle Z représente un groupement N-méthylepipéridyle ou tropyle ou quinuclidyle ou un groupement répondant à la formule générale II

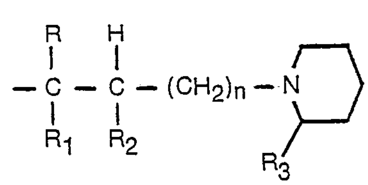

(II)

dans laquelle *n* peut être 0 ou 1, R représente un atome d'hydrogène, un groupement alcoyle ayant de 1 à 4 atomes de carbone ou un groupement phényle, $R_1$ représente un atome d'hydrogène ou un groupement alcoyle ayane de 1 à 4 atomes de carbone, $R_2$ représente un atome d'hydrogène ou un groupement hydroxy, ou bien R, $R_1$ et $R_2$, conjointement avec les atomes de carbone auxquels ils sont rattachés, représentent un groupement cycloalcoyle ayant de 4 à 6 atomes de carbone et $R_3$ représente un atome d'hydrogène ou un groupement alcoyle ayant de 1 à 4 atomes de carbone, avec la convention que R, $R_1$, $R_2$ et $R_3$ ne représentent jamais tous simultanément des atomes d'hydrogène; ou sel pharmaceutiquement acceptable de cet ester.

2. [(Méthyle-2 pipéridino)-3-propyle] carboxylate-8 méthyle-3 flavone.
3. (N-méthyle-pipéridyle-3) carboxylate-8 méthyle-3 flavone.
4. (Quinuclidyle-3) carboxylate-8 méthyle-3 flavone.
5. (Cis-piperidino-2 cyclohexyle) carboxylate-8 méthyle-3 flavone.
6. (Trans-pipéridino-2 cyclohexyle) carboxylate-8 méthyle-3 flavone.
7. (Diméthyle-1,1-pipéridino-2-éthyle) carboxylate-8 méthyle-3 flavone.
8. [Diméthyle-1,1-(méthyle-2 pipéridino)-2-éthyle] carboxylate-8 méthyle-3 flavone.
9. Tropylecarboxylate-8 méthyle-3 flavone.
10. (Hydroxy-2-pipéridino-3-propyle) carboxylate-8 méthyle-3 flavone.
11. (Méthyle-1-pipéridino-3-propyle) carboxylate-8 méthyle-3 flavone.
12. (Méthyle-1-pipéridyle-4) carboxylate-8 méthyle-3 flavone.
13. (Méthyle-1-pipéridino-2-éthyle) carboxylate-8 méthyle-3 flavone.
14. (Phényle-1-pipéridino-2-éthyle) carboxylate-8 méthyle-3 flavone.
15. (Pipéridino méthyle-2-cyclohexyle) carboxylate-8 méthyle-3 flavone.
16. Procédé de préparation d'un composé selon la revendication 1, consistant à condenser un halogénure de l'acide méthyle-3 flavone-carboxylique-8 répondant à la formule générale III

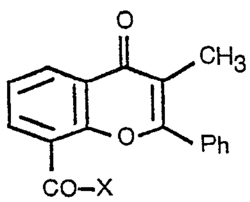

(III)

dans laquelle X représente un atome d'halogène, avec un alcool de formule générale ZOH dans laquelle Z est défini comme dans la revendication 1.

17. Procédé selon la revendication 16, dans lequel X représente un atome de chlore.
18. Procédé selon l'une des revendications 16 ou 17, dans lequel on effectue la réaction en chauffant l'alcool avec un excès du dérivé de la flavone, à une température comprise entre 140°C et 200°C, en l'absence de solvant.
19. Procédé selon l'une des revendications 16 ou 17, dans lequel on effectue la réaction de l'alcool avec une quantité équimoléculaire du dérivé de la flavone, en présence d'un solvant, à une température comprise entre 0°C et la température de reflux du solvant.
20. Procédé selon la revendication 4, caractérisé en ce que la réaction est effectuée en présence d'un agent accepteur d'acide.
21. Procédé de préparation d'un ester de l'acide méthyle-3 flavone-carboxylique-8 répondant à la formule générale I

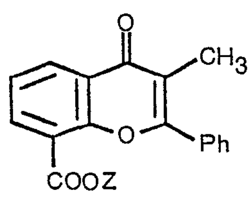

(I)

dans laquelle Z représente un groupement de formule générale II

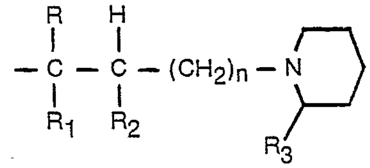

(II)

dans laquelle *n* prend la valeur 1, R et $R_1$ représentent, tous deux, des atomes d'hydrogène, $R_2$ représente un groupement hydroxy et $R_3$ représente un atome d'hydrogène ou un groupement alcoyle ayant de 1 à 4 atomes de carbone, ledit procédé consistant à faire réagir un composé répondant à la formule générale IV

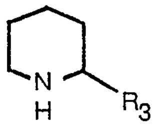

(IV)

dans laquelle $R_3$ est défini comme dans la revendication 1 avec 1'(époxy-2,3 propyle) carboxylate-8 méthyle-3 flavone en présence d'un catalyseur.

22. Procédé selon la revendication 21 dans lequel le catalyseur est la triéthylamine.

23. Procédé selon la revendication 22, dans lequel le réaction est effectuée dans un solvant, entre 20°C et 80°C, en utilisant des proportions équimoléculaires du dérivé de la flavone et du dérivé de la pipéridine.

24. Composition pharmaceutique contenant un ester de l'acide méthyle-3 flavone-carboxylique-8 selon la revendication 1, ou un sel pharmaceutiquement acceptable de cet ester, associé avec un excipient ou diluant pharmaceutiquement acceptable.

**Revendications pour l'Etat Contractant: AT**

1. Procédé de préparation d'un ester de l'acide méthyle-3-flavone-carboxylique-8 répondant à la formule générale I

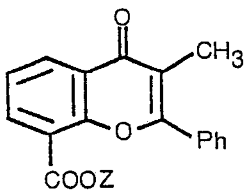

(I)

dans laquelle Z représente un groupement N-méthylepipéridyle ou tropÿle ou quinuclidyle ou un groupement répondant à la formule générale II

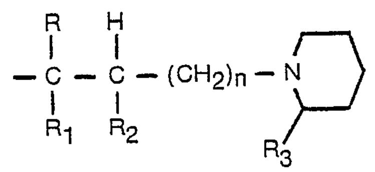

(II)

dans laquelle *n* peut être 0 ou 1, R représente un atome d'hydrogène, un groupement alcoyle ayant de 1 à 4 atomes de carbone ou un groupement phényle, $R_1$ représente un atome d'hydrogène ou un groupement alcoyle ayant de 1 à 4 atomes de carbone, $R_2$ représente un atome d'hydrogène ou un groupement hydroxy, ou bien R, $R_1$ et $R_2$, conjointement avec les atomes de carbone auxquels ils sont rattachés, représentent un groupement cycloalcoyle ayant de 4 à 6 atomes de carbone et $R_3$ représente un atome d'hydrogène ou un groupement alcoyle ayant de 1 à 4 atomes de carbone, avec la convention que R, $R_1$, $R_2$ et $R_3$ ne représentent jamais tous simultanément des atomes d'hydrogène, ledit procédé consistant à condenser un halogénure de l'acide méthyle-3 flavone-carboxylique-8 répondant à la formule générale III

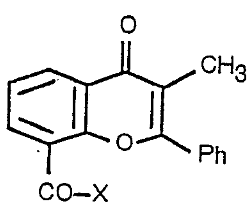

(III)

dans laquelle X représente un atome d'halogène, avec un alcool de formule générale ZOH dans laquelle Z est défini comme précédemment.

2. Procédé selon la revendication 1, dans lequel X représente un atome de chlore.

3. Procédé selon l'une des revendications 1 ou 2, dans lequel on effectue la réaction en chauffant l'alcool avec un excès du dérivé de la flavone, à une température comprise entre 140°C et 200°C, en l'absence de solvant.

4. Procédé selon l'une des revendications 1 ou 2, dans lequel on effectue la réaction de l'alcool avec une quantité équimoléculaire du dérivé de la flavone, en présence d'un solvant, à une température comprise entre 0°C et la température de reflux du solvant.

5. Procédé selon la revendication 4, dans lequel la réaction est effectuée en présence d'un agent accepteur d'acide.

6. Procédé de préparation d'un ester de l'acide méthyle-3 flavone-carboxylique-8 répondant à la formule générale I

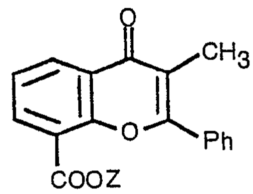

(I)

dans laquelle Z représente un groupement de formule générale II

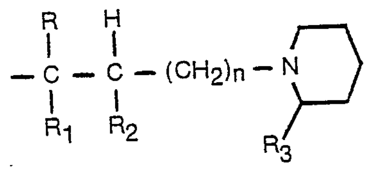

(II)

dans laquelle *n* prend la valeur 1, R et $R_1$ représentent, tous deux, des atomes d'hydrogène, $R_2$ représente un groupement hydroxy et $R_3$ représente un atome d'hydrogène ou un groupement alcoyle ayant de 1 à 4 atomes de carbone, ledit procédé consistant à faire réagir un composé répondant à la formule générale IV

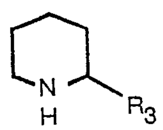

(IV)

dans laquelle $R_3$ est défini comme dans la revendication 1 avec 1'(époxy-2,3 propyle) carboxylate-8 méthyle-3 flavone en présence d'un catalyseur.

7. Procédé selon la revendication 6 dans lequel le catalyseur est la triéthylamine.

8. Procédé selon la revendication 7, dans lequel le réaction est effectuée dans un solvant, entre 20°C et 80°C, en utilisant des proportions équimoléculaires du dérivé de la flavone et du dérivé de la pipéridine.